(19) Europäisches Patentamt
European Patent Office
Office européen des brevets

(11) EP 2 345 456 B2

(12) # NOUVEAU FASCICULE DE BREVET EUROPEEN

Après la procédure d'opposition

(45) Date de publication et mention de la décision concernant l'opposition:
**09.12.2020 Bulletin 2020/50**

(45) Mention de la délivrance du brevet:
**03.05.2017 Bulletin 2017/18**

(21) Numéro de dépôt: **10191638.5**

(22) Date de dépôt: **18.11.2010**

(51) Int Cl.:
***A61K 8/49*** *(2006.01)* ***A61K 8/88*** *(2006.01)*
***A61Q 17/04*** *(2006.01)*

(54) **COMPOSITION FILTRANTE FLUIDE ANHYDRE COMPRENANT UNE PHASE HUILEUSE, UN FILTRE TRIAZINE PARTICULIER ET UN AGENT RHÉOLOGIQUE ÉPAISSISSANT OU GÉLIFIANT D'HUILE**

WASSERFREIE FLÜSSIGE FILTRIERENDE ZUBEREITUNG ENTHALTEND EINE ÖLIGE PHASE, EIN SPEZIFISCHES TRIAZINE-FILTER UND RHEOLOGISCH VERDICKENDE ODER GELIERENDE KOMPONENTE AUS EINEM ÖL

ANHYDROUS LIQUID SCREENING COMPOSITIONCONTAINING AN OILY PHASE, A SPECIFIC TRIAZINE FILTER AND AN OILY REOLOGICAL THICKENING OR GELIFYING AGENT.

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.12.2009 FR 0958876**

(43) Date de publication de la demande:
**20.07.2011 Bulletin 2011/29**

(73) Titulaire: **L'Oréal**
**75008 Paris (FR)**

(72) Inventeur: **Josso, Martin**
**75007, Paris (FR)**

(74) Mandataire: **Casalonga**
**Casalonga & Partners**
**Bayerstraße 71/73**
**80335 München (DE)**

(56) Documents cités:
**EP-A1- 0 904 770** **EP-A2- 1 216 690**
**WO-A1-03/013468** **WO-A1-03/053393**
**WO-A1-2007/068699** **FR-A1- 2 903 008**
**US-A1- 2005 065 251** **US-A1- 2006 292 095**
**US-A1- 2007 028 401** **US-A1- 2007 297 997**
**US-B2- 6 916 464**

- **DATABASE MINTEL [en ligne] Avril 2008 'Sport Clear Sun Spray' Database accession no. 894702**
- **ANDREAS DOMSCH: "Kieselsäure, Siliziumdioxid" In: "Die kosmetischen Pr parate", Band II,", 1992 vol. II, page 75, * , allgemeines Fachwissen, ***
- **ANDREAS DOMSCH: "3.3 Körperöle und -gele" In: "Die kosmetischen Präparate band II", 1994, XP055621440, vol. III, page 40, * , allgemeines Fachwissen, ***
- **RUDOLF VOIGT: "14.5.2 Plastibase" In: "Lehrbuch der pharmazeutischen Technologie", 1987 pages 269-271, 296, * , allgemeines Fachwissen, ***
- **FEY, OTTE: In: "Wörterbuch der Kosmetik", 1991 page 282, * , Stichwort Wachse, allgemeines Fachwissen, ***

Remarques:
Le dossier contient des informations techniques présentées postérieurement au dépôt de la demande et ne figurant pas dans le présent fascicule.

EP 2 345 456 B2

**Description**

La présente invention concerne une composition anhydre fluide comprenant dans un milieu cosmétiquement acceptable :

a) au moins une phase huileuse et
b) au moins un filtre UV triazine de formule (I) particulière et
c) au moins un agent rhéologique épaississant ou gélifiant de phase huileuse selon la revendication 1.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Dans le but d'assurer une protection de la peau et des matières kératiniques contre le rayonnement UV, on utilise généralement des compositions antisolaires comprenant des filtres organiques, actifs dans l'UV-A et actifs dans l'UV-B.

De nombreuses compositions cosmétiques destinées à la photoprotection (UV-A et/ou UV-B) de la peau ont été proposées à ce jour. On recherche tout particulièrement des formulations fluides qui assurent pour les utilisateurs une application facile sur la peau.

Parmi les compositions solaires fluides jusqu'alors proposées, les formulations anhydres type huile solaire sont particulièrement recherchées du fait de leur application facile et agréable sur la peau et de leur bonne résistance à l'eau. Cependant, elles sont peu répandues sur le marché des produits solaires du fait qu'il est difficile d'obtenir un facteur de protection solaire supérieur à 10 voire supérieur à 15 ainsi qu'un facteur de protection UVA répondant au ratio demandé par les différentes réglementations en matière de produits solaires, notamment supérieur à 5.

Le facteur de protection solaire (FPS) s'exprime mathématiquement par le rapport du temps d'irradiation nécessaire pour atteindre le seuil érythématogène avec le filtre UV au temps nécessaire pour atteindre le seuil érythématogène sans filtre UV. Il est évalué in vivo notamment selon la méthode Internationale publiée par Colipa / CTFA SA / JCIA (Mai 2006)

Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and efective of January 1, 1996).

Le facteur de protection $UVA_{PPD}$ (FP $UVA_{PPD}$) s'exprime mathématiquement par le rapport de la dose de rayonnement UV-A nécessaire pour atteindre le seuil de pigmentation avec le filtre UV (MPPDp) avec la dose de rayonnement UV-A nécessaire pour atteindre le seuil de pigmentation sans filtre UV (MPPDnp).

$$\text{FP UVA}_{PPD} = \frac{\text{MPPDp}}{\text{MPPDnp}}$$

En effet, une des difficultés principales rencontrées dans la fabrication des huiles solaires est qu'à la différence des autres types de formules solaires, une augmentation du taux de filtre solaire ne suffit pas à augmenter le facteur de protection

En effet, on connaît dans l'état de la technique des huiles solaires comprenant comme filtre organique lipophile la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V dans les demandes de brevet

WO9906014, EP845261, EP845260, EP821938, EP821940, EP800813, EP1300137, EP1027883. Ces huiles solaires présentent un FPS limité et l'augmentation du taux de filtre ne suffit pas pour l'augmenter de manière significative.

On a déjà proposé dans les demandes EP1813266 et EP2014277 d'utiliser dans des compositions solaires fluides aqueuses du type émulsion des polycondensats polyamides lipophiles en particulier un polymère poly(ester-amide) à terminaison ester (ETPEA) ou un polymère polyamide à terminaison amide tertiaire (ATPA) afin d'obtenir des facteurs de protection solaire élevés.

Cependant, l'ajout d'un tel polymère dans une huile solaire fluide contenant une quantité importante de filtres solaires peut entraîner une augmentation de la viscosité de la formulation, après fabrication et au cours du temps et notamment lors du stockage à basse température, ayant pour conséquence un faible écoulement de la composition et donc une utilisation difficile.

Il existe donc le besoin de trouver de nouvelles compositions solaires anhydres fluides, stables dans le temps permettant d'atteindre des facteurs de protection solaire plus élevés sans les inconvénients mentionnés ci-dessus.

Or, la Demanderesse vient maintenant de découvrir, de façon surprenante, que cet objectif pouvait être atteint avec une composition anhydre fluide comprenant dans un milieu cosmétiquement acceptable :

a) au moins une phase huileuse et
b) au moins un filtre UV triazine de formule (I) particulière que l'on définira en détail par la suite et
c) au moins un agent rhéologique épaississant ou gélifiant de phase huileuse choisi parmi :

(i) les polymères semi-cristallins issus d'un monomère à chaîne cristallisable choisi parmi les (méth)acrylates d'alkyle saturés en $C_{14}$ à $C_{22}$, et
(ii) les polycondensats polyamides lipophiles.

Cette découverte est à la base de la présente invention.

La présente invention concerne une composition anhydre fluide comprenant dans un milieu cosmétiquement acceptable :

a) au moins une phase huileuse et
b) au moins un filtre UV triazine de formule (I) particulière et
c) au moins un agent rhéologique épaississant ou gélifiant de phase huileuse tel que défini ci-avant.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par « composition fluide », on entend au sens de l'invention une composition ne se présentant pas sous une forme solide et dont la viscosité mesurée à l'aide d'un viscosimètre Rhéomat 180 à 25°C à la vitesse de rotation de 200 RPM après 30 secondes de rotation est inférieure à 0,5 Pa.s et plus préférentiellement inférieure à 0,2 Pa.s et plus particulièrement allant de 0,0001 Pa.s à 0,1 Pa.s.

On entend par « composition anhydre » une composition contenant moins de 1% en poids d'eau, voire moins de 0,5 % d'eau, et notamment exempte d'eau, l'eau n'étant pas ajoutée lors de la préparation de la composition mais correspondant à l'eau résiduelle apportée par les ingrédients mélangés.

Par "phase grasse liquide", au sens de la présente demande, on entend une phase grasse liquide à température ambiante (25°C) et pression atmosphérique (760mm de Hg), composée d'un ou plusieurs corps gras liquides à température ambiante, appelés aussi huiles, compatibles entre eux.

Par " lipophile" on entend tout composé cosmétique ou dermatologique susceptible d'être complètement dissous à l'état moléculaire dans une phase grasse liquide ou bien d'être solubilisé sous forme colloïdale (par exemple sous forme micellaire) dans une phase grasse liquide.

On entend par « huile » un corps gras liquide à la température ambiante (20 à 25°C).

On entend par "huile hydrocarbonée" toute huile comportant majoritairement des atomes de carbone et d'hydrogène, et éventuellement des groupements ester, éther, fluoré, acide carboxylique, alcool, silicone, amine, phényle et/ou aminoacide.

On entend par « agent rhéologique épaississant ou gélifiant de phase huileuse » un composé apte à augmenter la viscosité de la phase grasse de la composition ou à modifier ses propriétés rhéologiques tout en laissant celle-ci sous forme liquide.

**FILTRES UV TRIAZINE DE FORMULE (I)**

Les dérivés de 1,3,5-triazine conformes à l'invention répondent à la formule (I) suivante :

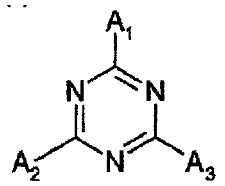

(I)

dans laquelle les radicaux $A_1$, $A_2$ et $A_3$, identiques ou différents désigne un groupe de formule (II) suivante :

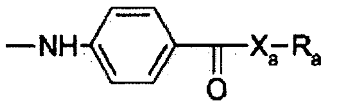

(II)

- un ou deux des groupements $X_a$-$R_a$ représentent le radical -NH-$R_a$ avec $R_a$ choisi parmi : un radical alkyle linéaire ou ramifié en $C_1$-$C_{18}$; un radical cycloalkyle en $C_5$-$C_{12}$ éventuellement substitué avec un ou plusieurs radicaux alkyles en $C_1$-$C_4$ ; un radical de formule (α), (β) ou (ε) suivantes :

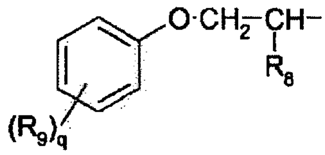

(α)

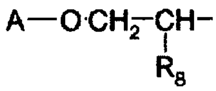

(β)

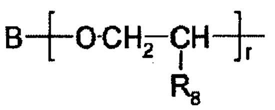

(ε)

dans lesquelles :

  - ◦ $R_8$ est l'hydrogène ou un radical alkyle en $C_1$-$C_4$ de préférence méthyle;
  - ◦ $R_9$ est alkyle en $C_1$-$C_4$ et de préférence méthyle ;
  - ◦ q est un nombre entier de 0 à 3;
  - ◦ r est un nombre entier de 1 à 10;
  - ◦ A est un radical alkyle en $C_4$-$C_8$ ou un radical cycloalkyle en $C_5$-$C_8$ ;
  - ◦ B est un radical alkyle en $C_1$-$C_4$ ;

- le ou les deux autres $X_a$-$R_a$ étant le radical -O-$R_a$ avec $R_a$, identiques ou différents choisis parmi : l'hydrogène; un métal alcalin; un radical ammonium éventuellement substitué par un ou plusieurs radicaux alkyles ou hydroxyalkyles ; un radical alkyle linéaire ou ramifié en $C_1$-$C_{18}$; un radical cycloalkyle en $C_5$-$C_{12}$ éventuellement substitué par un ou plusieurs radicaux alkyles en $C_1$-$C_4$ ; un radical de formule (α), (β) ou (ε) telles que définies ci-dessus.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante :

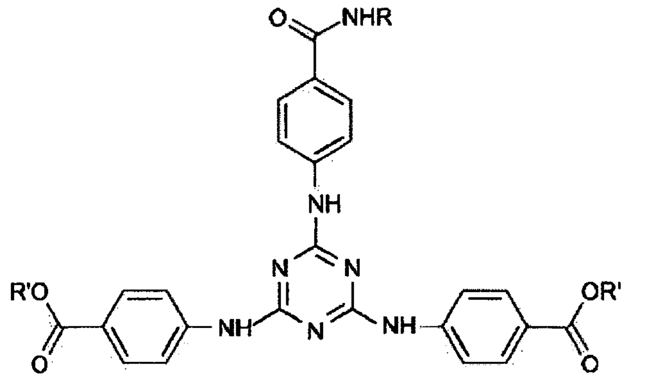

dans laquelle R' désigne un radical éthyl-2 hexyle et R désigne un radical tert-butyle.

Le composé triazine de formule (I) est de préférence présent dans les compositions selon l'invention dans des proportions allant de 0,1 à 10% en poids par rapport au poids total de la composition, et de préférence allant de 1 à 6% en poids par rapport au poids total de la composition.

## AGENT RHEOLOGIQUE EPAISSISSANT OU GELIFIANT DE PHASE GRASSE

L'agent rhéologique épaississant ou gélifiant de phase huileuse est de préférence présent en des quantités allant de 0.1% à 10%, plus préférentiellement de 1% à 7% et encore plus préférentiellement de 4% à 7% par rapport au poids total de la composition.

L'agent rhéologique épaississant ou gélifiant de phase huileuse est choisi parmi:

(i) les polymères semi-cristallins issus d'un monomère à chaîne cristallisable choisi parmi les (méth)acrylates d'alkyle saturés en $C_{14}$ à $C_{22}$, et
(ii) les polycondensats polyamides lipophiles.

### (1) Polymères semi-cristallins

On entend par "polymère semi-cristallin", des polymères comportant une partie cristallisable, chaîne pendante et/ou terminale cristallisable ou séquence cristallisable dans le squelette et/ou aux extrémités, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin selon l'invention a une température de fusion supérieure ou égale à 30 °C (notamment allant de 30 °C à 80 °C), de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquelles s'applique l'invention présentent une masse moléculaire moyenne en nombre supérieure ou égale à 1 000.

De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par

rapport au squelette du polymère. Une séquence est un groupement d'atomes appartenant au squelette, groupement constituant un des motifs répétitif du polymère.

De préférence, la ou les séquences ou chaînes cristallisables des polymères semi-cristallins représentent au moins 30 % du poids total de chaque polymère et mieux au moins 40 %. Les polymères semi-cristallins de l'invention à séquences cristallisables sont des polymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

Selon l'invention, le polymère est issu d'un monomère à chaîne cristallisable choisi parmi les (méth)acrylates d'alkyle saturés en $C_{14}$ à $C_{22}$ et encore plus particulièrement les poly(acrylate de stéaryle) ou les poly(acrylate de béhényle).

A titre d'exemple particulier de polymère semi-cristallin structurant utilisable dans la composition selon l'invention, on peut citer les polymères ayant pour nom INCI "POLY C10-30 ALKYL ACRYLATE comme les produits Intelimer® de la société AIR PRODUCTS comme le produit Intelimer® IPA 13-1 qui est un polyacrylate de stéaryle ou le produit Intelimer® IPA 13-6 qui est un polymère de béhényle.

**(2) Polycondensats polyamides lipophiles**

Par « polycondensat », on entend au sens de l'invention un polymère obtenu par polycondensation à savoir par réaction chimique entre des monomères possédant des groupes fonctionnels différents choisis en particulier parmi les fonctions acides, alcools et amines.

Par « polymère », on entend au sens de l'invention un composé ayant au moins 2 motifs de répétition, de préférence au moins 3 motifs de répétition et mieux encore 10 motifs de répétition.

Le ou les polycondensats polyamides lipophiles sont de préférence présents dans les compositions de l'invention dans des concentrations allant de 0,1 à 15% en poids par rapport au poids total de la composition, plus préférentiellement de 1 à 8 % en poids.

Les polycondensats polyamides lipophiles peuvent être notamment choisis parmi les polymères de polyamide comportant a) un squelette polymérique ayant des motifs de répétition hydrocarbonés pourvus d'au moins un motif amide non pendant, et éventuellement b) au moins une chaîne grasse pendante et/ou au moins une chaîne grasse terminale éventuellement fonctionnalisées, comprenant au moins 4 atomes de carbone et étant liées à ces motifs hydrocarbonés.

Par "chaînes fonctionnalisées" au sens de l'invention, on entend une chaîne alkyle comportant un ou plusieurs groupes fonctionnels ou réactifs notamment choisis parmi les groupes amides, hydroxyle, éther, oxyalkylène ou polyoxyalkylène, halogène, dont les groupes fluorés ou perfluorés, ester, siloxane, polysiloxane. En outre, les atomes d'hydrogène d'une ou plusieurs chaînes grasses peuvent être substitués au moins partiellement par des atomes de fluor.

Par "motifs de répétition hydrocarbonés", on entend au sens de l'invention un motif comportant de 2 à 80 atomes de carbone, et de préférence de 2 à 60 atomes de carbone, portant des atomes d'hydrogène et éventuellement des atomes d'oxygène, qui peut être linéaire, ramifié ou cyclique, saturé ou insaturé. Ces motifs comprennent, en outre, chacun au moins un groupement amide avantageusement non pendants et se trouvant dans le squelette polymérique.

Avantageusement, les chaînes pendantes sont liées directement à l'un au moins des atomes d'azote du squelette polymérique.

Le polycondensat polyamide lipophile peut comprendre entre les motifs hydrocarbonés des motifs siliconés ou des motifs oxyalkylénés.

En outre, le polycondensat polyamide lipophile de la composition de l'invention comprend avantageusement de 40 à 98 % de chaînes grasses par rapport au nombre total des motifs amide et des chaînes grasses et mieux de 50 à 95 %.

De préférence, les chaînes grasses pendantes sont liées à l'un au moins des atomes d'azote des motifs amide du polymère. En particulier, les chaînes grasses de ce polyamide représentent de 40 à 98 % du nombre total des motifs amide et des chaînes grasses, et mieux de 50 à 95 %.

Avantageusement, le polycondensat polyamide lipophile présente une masse moléculaire moyenne en poids inférieure à 100 000 (notamment allant de 1000 à 100 000), en particulier inférieure à 50 000 (notamment allant de 1000 à 50 000), et plus particulièrement allant de 1000 à 30 000, de préférence de 2000 à 20 000, et mieux de 2000 à 10 000.

Le polycondensat polyamide lipophile est non soluble dans l'eau, notamment à 25 °C. En particulier, il ne comporte pas de groupe ionique.

Comme polycondensats polyamides lipophiles préférés utilisables dans l'invention, on peut citer les polyamides ramifiés par des chaînes grasses pendantes et/ou des chaînes grasses terminales ayant de 6 à 120 atomes de carbone et mieux de 8 à 120 et notamment de 12 à 68 atomes de carbone, chaque chaîne grasse terminale étant liée au squelette polyamide par au moins un groupe de liaison L. Le groupe de liaison L peut être choisi parmi les groupes ester, éther,

amine, urée, uréthane, thioester, thioéther, thiurée, thiouréthane. De préférence, ces polymères comportent une chaîne grasse à chaque extrémité du squelette polyamide.

Ces polymères sont de préférence des polymères résultant d'une polycondensation entre un diacide carboxylique ayant au moins 32 atomes de carbone (ayant notamment de 32 à 44 atomes de carbone) avec une amine choisie parmi les diamines ayant au moins 2 atomes de carbone (notamment de 2 à 36 atomes de carbone) et les triamines ayant au moins 2 atomes de carbone (notamment de 2 à 36 atomes de carbone). Le diacide est de préférence un dimère issu d'acide gras à insaturation éthylénique ayant au moins 16 atomes de carbone, de préférence de 16 à 24 atomes de carbone, comme l'acide oléique, linoléique ou linolénique. La diamine est de préférence l'éthylène diamine, l'hexylène diamine, l'hexaméthylène diamine. La triamine est par exemple l'éthylène triamine. Pour les polymères comportant un ou 2 groupements d'acide carboxylique terminaux, il est avantageux de les estérifier par un monoalcool ayant au moins 4 atomes de carbone, de préférence de 10 à 36 atomes de carbone et mieux de 12 à 24 et encore mieux de 16 à 24, par exemple 18 atomes de carbone.

Le polycondensat polyamide lipophile de la composition selon l'invention peut être en particulier choisi parmi les polymères de formule (V) suivante :

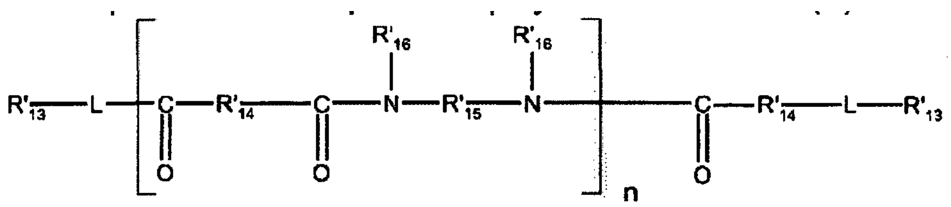

(V)

dans laquelle :

n est un nombre entier allant de1 à 30,

$R'_{13}$ représente à chaque occurrence indépendamment une chaîne grasse et est choisi parmi un groupe alkyle ou alcényle ayant au moins 1 atome de carbone et notamment de 4 à 24 atomes de carbone ;

$R'_{14}$ représente à chaque occurrence indépendamment un radical hydrocarboné comprenant de 1 à 52 atomes de carbone ;

$R'_{15}$ représente à chaque occurrence indépendamment un groupement organique comprenant au moins un atome choisi parmi les atomes de carbone, d'hydrogène, d'azote, à la condition que $R'_{15}$ comprend au moins 3 atomes de carbone ;

$R'_{16}$ représente à chaque occurrence indépendamment : un atome d'hydrogène, un groupes alkyle en comprenant de 1 à 10 atomes de carbone, ou une liaison directe à au moins un groupe choisi parmi $R'_{15}$ et un autre $R'_{16}$ de sorte que quand ledit groupe est un autre $R'_{16}$, l'atome d'azote auquel sont liés à la fois $R'_{15}$ et $R'_{16}$ fasse partie d'une structure hétérocyclique définie par $R'_{16}$-N-$R'_{15}$, à la condition qu'au moins 50% des $R'_4$ représentent un atome d'hydrogène, et

L représente un groupe de liaison de préférence choisi parmi ester, éther, amine, urée, uréthane, thioester, thioéther, thiurée, thiouréthane, éventuellement substitué par au moins un groupe $R'_1$ tel que défini ci-dessus.

Selon un mode de réalisation, ces polymères sont choisis parmi les polymères de formule (V) dans laquelle le groupe de liaison L représente un groupe ester

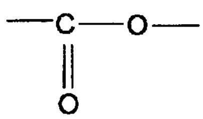

Ces polymères sont plus spécialement ceux décrits dans le document US-A-5783657 de la société Union Camp.

Chacun de ces polymères satisfait notamment à la formule (B) suivante :

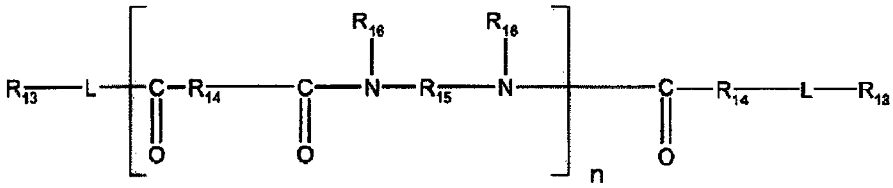

(VI)

dans laquelle :

- m désigne un nombre entier de motifs amide tel que le nombre de groupes ester représente de 10 % à 50 % du nombre total des groupes ester et amide ;
- $R_{13}$ est à chaque occurrence indépendamment un groupe alkyle ou alcényle ayant au moins 4 atomes de carbone et notamment de 4 à 24 atomes de carbone ;
- $R_{14}$ représente à chaque occurrence indépendamment un groupe hydrocarboné en $C_4$ à $C_{42}$ à condition que 50 % des groupes $R_{14}$ représentent un groupe hydrocarboné en C30 à C42 ;
- $R_{15}$ représente à chaque occurrence indépendamment un groupe organique pourvu d'au moins 2 atomes de carbone, d'atomes d'hydrogène et optionnellement d'un ou plusieurs atomes d'oxygène ou d'azote ;
- et $R_{16}$ représente à chaque occurrence indépendamment un atome d'hydrogène, un groupe alkyle en $C_1$ à $C_{10}$ ou une liaison directe à $R_{15}$ ou à un autre $R_{16}$ de sorte que l'atome d'azote auquel sont liés à la fois $R_3$ et $R_4$ fasse partie d'une structure hétérocyclique définie par $R_{16}$-N-$R_{15}$, avec au moins 50 % des $R_4$ représentant un atome d'hydrogène.

Dans le cas particulier de la formule (VI), les chaînes grasses terminales éventuellement fonctionnalisées au sens de l'invention sont des chaînes terminales liées au dernier atome d'azote, du squelette polyamide.

En particulier, les groupes ester de la formule (VI), qui font partie des chaînes grasses terminales et/ou pendantes au sens de l'invention, représentent de 15 à 40 % du nombre total des groupes ester et amide et mieux de 20 à 35 %.

De plus, m représente avantageusement un nombre entier allant de 1 à 5 et mieux supérieur à 2.

De préférence, $R_{13}$ est un groupe alkyle en $C_{12}$ à $C_{22}$ et de préférence en $C_{16}$ à $C_{22}$. Avantageusement, $R_{14}$ peut être un groupe hydrocarboné (alkylène) en $C_{10}$ à $C_{42}$. De préférence, 50 % au moins et mieux au moins 75 % des $R_2$ sont des groupes ayant de 30 à 42 atomes de carbone. Les autres $R_2$ sont des groupes hydrogénés en $C_4$ à $C_{19}$ et même en $C_4$ à $C_{12}$.

De préférence, $R_{15}$ représente un groupe hydrocarboné en $C_2$ à $C_{36}$ ou un groupe polyoxyalkyléné et $R_{16}$ représente un atome d'hydrogène. De préférence, $R_{15}$ représente un groupe hydrocarboné en $C_2$ à $C_{12}$.

Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.

En général, les polymères de formule (VI) se présentent sous forme de mélanges de polymères, ces mélanges pouvant en outre contenir un produit de synthèse correspondant à un composé de formule (VI) où n vaut 0, c'est-à-dire un diester.

Selon une forme particulièrement préférée de l'invention, on utilisera un mélange de copolymères d'un diacide en $C_{36}$ condensé sur l'éthylène diamine ; les groupes ester terminaux résultent de l'estérification des terminaisons d'acide restantes par l'alcool cétylique, stéarylique ou leurs mélanges (appelés aussi alcool cétylstéarylique) (nom INCI: ETHYLENEDIAMINE/STEARYL DIMER DILINOLEATE COPOLYMER). Sa masse moléculaire moyenne en poids est de préférence de 6000. Ces mélanges sont notamment vendus par la société ARIZONA CHEMICAL sous les noms commerciaux UNICLEAR 80 et UNICLEAR 100 VG respectivement sous forme de gel à 80 % (en matière active) dans une huile minérale et à 100 % (en matière active). Ils ont un point de ramollissement de 88°C à 94°C.

A titre de polycondensats polyamides répondant à la formule générale (VI), on peut également citer les polymères comprenant au moins une chaîne grasse terminale liée au squelette polymérique par au moins un groupe de liaison amide tertiaire (appelés aussi amide terminated polyamide ou ATPA). Pour plus d'information sur ces polymères, on peut se référer au document US 6503522.

Selon une forme particulièrement préférée de l'invention, on utilisera plus particulièrement un copolymère de diacide linoléique hydrogéné, d'éthylènediamine, de di($C_{14}$-$C_{18}$)alkylamine(s) (nom INCI: ETHYLENEDIAMIDE/HYDROGENATED DIMER DILINOLEATE COPOLYMER BIS-DI-$C_{14}$-$C_{18}$ ALKYL AMIDE). Ce copolymère est notamment vendu sous le nom commercial SYLVACLEAR A200V par la société ARIZONA CHEMICAL.

Selon un autre mode de réalisation, le polyamide de formule (A) peut également être un poly(ester-amide) à extrémités ester (ester-terminated poly(ester-amide) ou ETPEA), comme par exemple ceux dont la préparation est décrite dans le document US 6 552 160.

Selon une forme particulièrement préférée de l'invention, on utilisera plus particulièrement un copolymère de

diacide linoléique hydrogéné, d'éthylènediamine, de néopentylglycol et d'alcool stéarylique (nom INCI : BIS-STEARYL ETHYLENEDIAMINE/NEOPENTYL GLYCOL/STEARYL HYDROGENATED DIMER DILINOLEATE COPOLYMER). Ce copolymère est notamment vendu sous le nom commercial SYLVACLEAR C75 V par la société ARIZONA CHEMICAL.

Comme polycondensats polyamides utilisables dans l'invention, on peut encore citer ceux comprenant au moins une chaîne grasse terminale liée au squelette polymérique par au moins un groupe de liaison éther ou polyéther (il est dit alors éther terminated poly(éther)amide). De tels polymères sont décrits par exemple dans le document US 6 399 713.

Les polyamides conformes à l'invention ont avantageusement une température de ramollissement supérieure à 65°C et pouvant aller jusqu'à 190°C. De préférence, il présente une température de ramollissement allant de 70 à 130°C et mieux de 80 à 105°C. Le polyamide est en particulier un polymère non cireux.

Comme polycondensats polyamides utilisables dans l'invention, on peut aussi citer les résines polyamides résultant de la condensation d'un acide dicarboxylique aliphatique et d'une diamine (incluant les composés ayant plus de 2 groupes carbonyle et 2 groupes amine), les groupes carbonyle et amine de motifs unitaires adjacents étant condensés par une liaison amide. Ces résines polyamides sont notamment celles commercialisées sous la marque Versamid® par les sociétés General Mills, Inc. et Henkel Corp. (Versamid 930, 744 ou 1655) ou par la société Olin Mathieson Chemical Corp., sous la marque Onamid® notamment Onamid S ou C. Ces résines ont une masse moléculaire moyenne en poids allant de 6000 à 9000. Pour plus d'information sur ces polyamides, on peut se référer aux documents US 3645705 et US 3148125. Plus spécialement, on utilise les Versamid® 930 ou 744.

On peut aussi utiliser les polyamides vendus par la société Arizona Chemical sous les références Uni-Rez (2658, 2931, 2970, 2621, 2613, 2624, 2665, 1554, 2623, 2662) et le produit vendu sous la référence Macromelt 6212 par la société Henkel. Pour plus d'information sur ces polyamides, on peut se référer au document US 5500209.

Il est aussi possible d'utiliser des résines de polyamides issues de légumes comme celles décrites dans les brevets US 5783657 et US 5998570.

Parmi ces agents épaississants, on utilisera encore plus préférentiellement les polycondensats polyamides lipophiles.

## PHASE HUILEUSE

La phase huileuse conforme à l'invention contient en général au moins une huile hydrocarbonée volatile ou non-volatile et/ou une huile siliconée, volatile ou non-volatile,.

Par " huile volatile", on entend au sens de l'invention une huile susceptible de s'évaporer au contact de la peau ou de la fibre kératinique en moins d'une heure, à température ambiante et pression atmosphérique. Le ou les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

La phase huileuse représente de préférence une quantité de 30 à 99,8% en poids et plus préférentiellement de 40 à 90% en poids par rapport au poids total de la composition.

### a) Huiles hydrocarbonées

Comme huiles hydrocarbonées non volatiles utilisables selon l'invention, on peut notamment citer :

(i) les huiles hydrocarbonées d'origine végétale telles que les triesters de glycérides qui sont en général des triesters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamlt Nobel,

(ii) les éthers de synthèse ayant de 10 à 40 atomes de carbone ;

(iii) les hydrocarbures linéaires ou ramifiés, d'origine minérale ou synthétique tels que la vaseline, les polydécènes, le polyisobutène hydrogéné tel que le parléam, le squalane, et leurs mélanges ;

(iv) les esters de synthèse comme les huiles de formule RCOOR' dans laquelle R représente le reste d'un acide gras linéaire ou ramifié comportant de 1 à 40 atomes de carbone et R' représente une chaîne hydrocarbonée notamment ramifiée contenant de 1 à 40 atomes de carbone à condition que R + R' soit ≥ 10, comme par exemple l'huile de Purcellin (octanoate de cétostéaryle), le myristate d'isopropyle, le palmitate d'isopropyle, le benzoate d'alcools en $C_{12}$-$C_{15}$ comme le produit vendu sous la dénomination commerciale « FINSOLV TN » ou « WITCONOL TN » par la société WITCO ou « TEGOSOFT TN » par la société EVONIK GOLDSCHMIDT , le Benzoate de 2-éthylphenyle comme le produit commercial vendu sous le nom « X-TEND 226» par la société ISP, le lanolate d'isopropyle, le laurate d'hexyle, l'adipate de diisopropyle, l'isononanoate d'isononyle, l'érucate d'oléyle, le palmitate de 2-éthyl-hexyle, l'isostéarate d'isostéaryle, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ; les citrates ou tartrates comme les tartrates de di-alkyle linéaire en $C_{12}$-$C_{13}$ tels que ceux vendus sous le nom COSMACOL ETI par la Société ENICHEM AUGUSTA INDUSTRIALE ainsi que les tartrates de di-alkyle linéaire en $C_{14}$-$C_{15}$ tels que ceux vendus sous le nom COSMACOL ETL par la même société ; les acétates.

(v) les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;

(vi) les acides gras supérieurs tels que l'acide oléique, l'acide linoléique, l'acide linolénique ;

(vii) les carbonates comme le dicaprylyl carbonate comme le produit vendu sous la dénomination « CETIOL CC » par la société COGNIS ;

(viii) les amides grasses comme l'Isopropyl N-lauroyl sarcosinate comme le produit vendu sous le nom commercial ELDEW SL205" de chez AJINOMOTO et leurs mélanges.

Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, les alcanes décrits dans les demandes de brevets de la société Cognis WO 2007/068371, ou WO20081155059 (mélanges d'alcanes distincts et différant d'au moins un carbone). Ces alcanes sont obtenus à partir d'alcools gras, eux-mêmes obtenus à partir d'huile de coprah ou de palme, les huiles vendues sous les noms commerciaux d'Isopars ou de Permetyls, les esters ramifiés en $C_8$-$C_{16}$ le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées.

Selon un mode de réalisation, le solvant volatil est choisi parmi les huiles volatiles hydrocarbonées ayant de 8 à 16 atomes de carbone et leurs mélanges.

Parmi les huiles hydrocarbonées utilisables selon l'invention, on préférera plus particulièrement les triesters de glycéride et notamment les triglycérides des acides caprylique/caprique, les esters de synthèse et notamment l'isononanoate d'isononyle, l'érucate d'oléyle, le benzoate d'alcools en $C_{12}$-$C_{15}$ et les alcools gras notamment l'octyldodécanol.

**b) les huiles siliconées**

Les huiles siliconées non volatiles peuvent être choisies notamment parmi les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Comme huiles volatiles siliconées, on peut citer par exemple les huiles de silicones linéaires ou cycliques volatiles, notamment celles ayant une viscosité ≥ 8 centistokes ($8\ 10^{-6}\ m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl

trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I) :

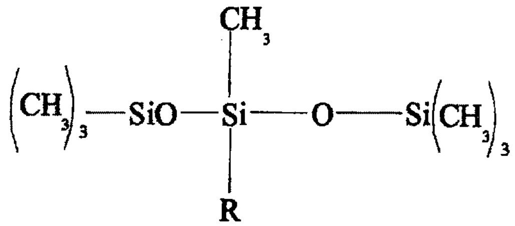

où R représente un groupe alkyle comprenant de 2 à 4 atomes de carbone et dont un ou plusieurs atomes d'hydrogène peuvent être substitués par un atome de fluor ou de chlore.

Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

**MONOALCOOL EN $C_1$-$C_3$ LINEAIRE**

Selon une forme particulièrement préférée de l'invention, les compopsitions de l'invention contiennent en outre au moins un mono-alcool en $C_1$-$C_3$ choisi parmi le méthanol, l'éthanol, le propanol ou leurs mélanges. On choisira plus particulièrement l'éthanol.

Le ou ledit monoalcool sont de préférence présents à des concentrations allant de 0,1 à 40% en poids, plus préférentiellement de 2 à 10 % en poids par rapport au poids total de la composition.

**FILTRES LIPOPHILES ADDITIONNELS**

Selon une forme particulièrement préférée de l'invention, les compopsitions de l'invention contiennent en outre au moins un filtre organique lipophile notamment choisi parmi les dérivés de l'acide para-aminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine autres que ceux de formule (I), les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

Parmi les filtres UVA organiques lipophiles capables d'absorber les UV de 320 à 400 nm, on peut citer

Les dérivés du dibenzoylméthane :

- le 4-isopropyl-dibenzoylméthane, vendu sous la dénomination de "EUSOLEX 8020" par la Société MERCK, et répondant à la formule suivante :

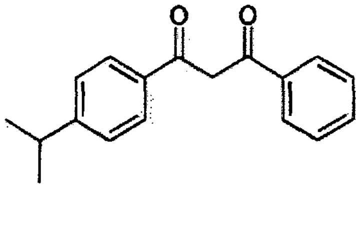

- le 1-(4-methoxy-1-benzofuran-5-yl)-3-phenylpropane-1,3-dione, proposé à la vente par la société QUEST sous le

nom de Pongamol de formule :

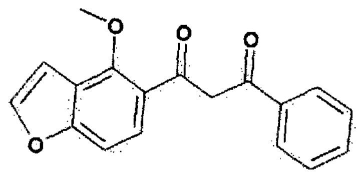

- le 1-(4-tert-butylphenyl)-3-(2-hydroxyphenyl)propane-1,3-dione de formule :

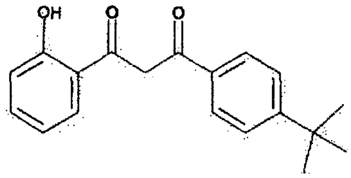

- le Butyl Methoxydibenzoylmethane vendu notamment sous le nom commercial « PARSOL 1789 » par HOFFMANN LA ROCHE,

Les aminobenzophénones

2-(4-diethylamino-2-hydroxybenzoyl)-benzoate de n-hexyle vendu sous le nom commercial« UVINUL A +»

Les dérivés anthraniliques :

Menthyl anthranilate vendu sous le nom commercial « NEO HELIOPAN MA » par HAARMANN et REIMER,

Les dérivés de 4,4-diarylbutadiène :

1,1-dicarboxy (2,2'-diméthyl-propyl)-4,4-diphénylbutadiène
Les préférentiels sont :

Butyl Methoxydibenzoylmethane 2-(4-diethylamino-2-hydroxybenzoyl)-benzoate de n-hexyle

Parmi les filtres UVB organiques lipophiles capables d'absorber les UV de 280 à 320 nm, on peut citer

Les para-aminobenzoates :

Ethyl PABA
Ethyl Dihydroxypropyl PABA
Ethylhexyl Dimethyl PABA (ESCALOL 507 de ISP)

Les dérivés salicyliques :

- Homosalate vendu sous le nom « Eusolex HMS » par Rona/EM Industries,
- Ethylhexyl Salicylate vendu sous le nom « NEO HELIOPAN OS » par HAARMANN et REIMER,
- Dipropyleneglycol Salicylate vendu sous le nom « DIPSAL » par SCHER, TEA Salicylate, vendu sous le nom « NEO HELIOPAN TS » par Haarmann et REIMER,

Les cinnamates

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Diisopropyl Methylcinnamate,
Cinoxate,
Glyceryl Ethylhexanoate Diméthoxycinnamate

Les dérivés de β,β'-diphénylacrylate :

Octocrylène, vendu notamment sous le nom commercial « UVINUL N539 » par BASF,
Etocrylène, vendu notamment sous le nom commercial « UVINUL N35 » par BASF,

Les dérivés du benzylidène camphre :

3-Benzylidène camphre fabriqué sous le nom « MEXORYL SD » par CHIMEX, Méthylbenzylidène camphre vendu sous le nom « EUSOLEX 6300 » par MERCK,
Polyacrylamidométhyle Benzylidène Camphre fabriqué sous le nom « MEXORYL SW » par CHIMEX,

Les dérivés de triazine :

Ethylhexyl triazone vendu notamment sous le nom commercial « UVINUL T150 » par BASF,
2,4,6-tris(4'-amino benzalmalonate de dinéopentyle)-s-triazine
la 2,4,6-tris-(4'-amino benzalmalonate de diisobutyle)-s- triazine,
2,4-bis(4'-amino benzalmalonate de dinéopentyle)-6-(4'-aminobenzoate de n-butyle)-s-triazine,
2,4-bis(4'-amino benzoate de n-butyle)-6-(aminopropyltrisiloxane)-s-triazine,

Les dérivés d'imidazolines :

Ethylhexyl Dimethoxybenzylidene Dioxoimidazoline Propionate,

Les dérivés du benzalmalonate :

Polyorganosiloxanes à fonction benzalmalonate tels que le Polysilicone-15 vendu sous la dénomination commerciale « PARSOL SLX » par HOFFMANN LA ROCHE
Di-neopentyl 4'-méthoxybenzalmalonate,

Les dérivés de mérocyanine

Octyl-5-N,N-diethylamino-2-phenysulfonyl-2,4-pentadienoate

Les préférentiels sont :

Homosalate
Ethylhexyl Salicylate
Ethylhexyl Methoxycinnamate
Octocrylène
Ethylhexyl triazone
2,4-bis(4'-amino benzoate de n-butyle)-6-(aminopropyltrisiloxane)-s-triazine, Octyl-5-N,N-diethylamino-2-phenysulfonyl-2,4-pentadienoate

Parmi les filtres organiques lipophiles à spectre large capables d'absorber les UVA et les UVB, on peut citer

Les dérivés de benzophénone

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-5,
Benzophenone-6 vendu sous le nom commercial « Helisorb 11 » par Norquay Benzophenone-8 vendu sous le nom commercial « Spectra-Sorb UV-24 » par American Cyanamid
Benzophenone-10,
Benzophenone-11,
Benzophenone-12,

Dérivés de benzotriazole :

Drometrizole Trisiloxane vendu sous le nom « Silatrizole » par RHODIA CHIMIE
Bumetrizole vendu sous le nom TINOGUARD AS par CIBA-GEIGY

Les dérivés bis-résorcinyl triazines

Bis-Ethylhexyloxyphénol Méthoxyphenyl Triazine vendu sous le nom commercial « TINOSORB S » par CIBA GEIGY,

Les dérivés de benzoxazole :

2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V

Les préférentiels sont :

Benzophenone-3
Drometrizole Trisiloxane
Bis-Ethylhexyloxyphénol Méthoxyphenyl Triazine

Les filtres organiques lipophiles sont généralement présents dans les compositions selon l'invention dans des proportions allant de 0,1 à 50% en poids par rapport au poids total de la composition, et de préférence allant de 2 à 30% en poids par rapport au poids total de la composition.
Selon une forme particulière de l'invention, les compositions seront transparentes et présenteront de préférence une turbidité inférieure à 1000 NTU (Unités de Turbidité Nephélométique) à 25°C, de préférence inférieure à 50 NTU à

25°C et encore plus préférentiellement inférieure à 15 NTU, mesurée avec un appareil 2100P Turbidimeter de la société HACH. (à vérifier)

Selon une forme particulièrement préférée de l'invention, les compositions présenteront un FPS supérieur à 10 voire supérieur à 15 et même supérieur à 20.

Selon une forme particulièrement préférée de l'invention, les compositions présenteront un FP $UVA_{PPD}$ supérieur à 5, elles respectent aussi la réglementation notamment européenne qui veut que le ratio SPF /PPD soit inférieur à 3.

## ADDITIFS

La composition huileuse du produit de l'invention peut également contenir différents additifs qui peuvent être solubles dans la phase huileuse, ou être en dispersion dans ladite phase huileuse notamment choisis parmi les colorants lipophiles, les actifs lipophiles, les solvants organiques, les conservateurs, les agents insectifuges, les huiles essentielles, les parfums, les émollients, les propulseurs.

Parmi les actifs cosmétiques lipophiles, on peut citer par exemple les antioxydants, les agents kératolytiques tels que les acides N-alkyl salicyliques, par exemple l'acide N-octanoyl-5 salicylique ; les vitamines comme la vitamine E (tocophérol et dérivés), la vitamine A (rétinol et dérivés) ; les adoucissants et tout actif lipophile habituellement utilisé dans le soin de la peau ou des cheveux.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Un autre objet de la présente invention est constitué par l'utilisation des compositions selon l'invention telles que définies ci-dessus comme produit pour le soin cosmétique et/ou de maquillage de la peau, des ongles, des cheveux, des cils, sourcils et/ou du cuir chevelu, notamment comme produit de soin, produit de protection solaire.

Les compositions cosmétiques selon l'invention peuvent par exemple être utilisées comme produit de consistance liquide de soin et/ou de protection solaire et /ou de photoprotection quotidienne et/ou de maquillage pour le visage et/ou le corps et/ou les cheveux.

## AGENTS COLORANTS ADDITIONNELS

Selon une autre forme particulière de l'invention, les compositions de l'invention peuvent comporter en plus un ou plusieurs agents de coloration additionnels.

Les agents de coloration additionnels peuvent être également choisis parmi les colorants directs synthétiques ou naturels. Il peut s'agir de colorants organiques ou minéraux.

Les colorants organiques liposolubles, synthétiques ou naturels sont, par exemple, le DC Red 17, le DC Red 21, le DC Red 27, le DC Green 6, le DC Yellow 11, le DC Violet 2, le DC Orange 5, le rouge Soudan, les carotènes (le β-carotène, le lycopène), les xanthophylles (capsanthine, capsorubine, lutéine), l'huile de palme, le brun Soudan, le jaune quinoléine, le rocou, le curcumin.

Les agents de coloration additionnels peuvent être également choisis parmi les matières colorantes particulaires qui sont choisies de préférence parmi les pigments, les nacres ou pigments interférentiels, les paillettes.

Par pigments, il faut comprendre des particules de toute forme, blanches ou colorées, minérales ou organiques, insolubles dans le milieu physiologique, destinées à colorer la composition.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Au sens de la présente invention, l'expression "particules interférentielles ou nacres" désigne toute particule possédant généralement une structure multicouche telle qu'elle permette la création d'un effet de couleur par interférence des rayons lumineux qui diffractent et diffusent différemment selon la nature des couches. Les effets colorants obtenus sont liés à la structure lamellaire de ces particules et dérivent des lois physiques de l'optique des couches minces (voir : Pearl Lustre Pigments - Physical principles, properties, applications - R. Maisch, M. Weigand. Verlag Moderne Industrie). Ainsi, ces particules peuvent présenter des couleurs variant selon l'angle d'observation et l'incidence de la lumière.

Au sens de la présente invention, une structure multicouche entend désigner indifféremment une structure formée d'un substrat recouvert d'une unique couche ou une structure formée d'un substrat recouvert d'au moins deux voire de plusieurs couches consécutives.

La structure multicouche peut ainsi comporter une voire au moins deux couches, chaque couche indépendamment ou non de la (ou les) autre(s) couche(s) étant réalisée(s) en au moins un matériau choisi dans le groupe constitué par les matériaux suivants : $MgF_2$, $CeF_3$, ZnS, ZnSe, Si, $SiO_2$, Ge, Te, $Fe_2O_3$, Pt, Va, $Al2O_3$, MgO, $Y2O_3$, $S_2O_3$, SiO, $HfO_2$, $ZrO_2$, $CeO_2$, $Nb2O_5$, $Ta_2O_5$, TiO2, Ag, Al, Au, Cu, Rb, Ti, Ta, W, Zn, MoS2, cryolithe, alliages, polymères et leurs associations.

Généralement, la structure multicouche est de nature inorganique.

Plus particulièrement, les particules interférentielles considérées selon l'invention peuvent être des pigments interférentiels, ou encore des nacres naturelles ou synthétiques, monocouches ou multicouches, en particulier formées d'un substrat naturel à base, entre autres, de mica et qui est recouvert d'une ou plusieurs couches d'oxyde métallique.

Les particules interférentielles selon l'invention sont caractérisées en ce que 50% de la population massique a un diamètre (d50) inférieur à 40 $\mu$m, plus particulièrement inférieur à 30 $\mu$m, notamment inférieur à 20 $\mu$m, et en particulier inférieur à 15 $\mu$m, mesurée par un granulomètre laser, tel que par exemple le Mastersizer 2000® de Malvernet ou le BI90 +® de Broockhaven Instrument Corporation.

Conviennent tout particulièrement à l'invention, les nacres de type mica/oxyde d'étain/oxyde de titane telles que par exemple celles commercialisées sous les dénominations TIMIRON SILK BLUE®, TIMIRON SILK RED®, TIMIRON SILK GREEN®, TIMIRON SILK GOLD® et TIMIRON SUPER SILK® proposées par la société MERCK et les nacres mica/oxyde de fer/oxyde de titane telles que par exemple les FLAMENCO SATIN BLUE®, FLAMENCO SATIN RED® et FLAMENCO SATIN VIOLET® et FLAMENCO ORANGE 320C proposées par la société ENGELHARD et leurs mélanges.

Plus précisément, ces pigments peuvent être présents dans des quantités allant de 0,01 à 10% en poids et de préférence allant de 0,1 à 5% en poids par rapport au poids total de la composition.

## COMPOSITIONS VAPORISABLES

Les compositions selon l'invention peuvent se présenter sous forme d'huile vaporisable appliquée sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Ces compositions peuvent être aussi imprégnées sur des supports type lingettes, ou elles peuvent être conditionnées comme des lotions en flacon avec un réducteur.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### Exemple 1 FPS vivo = 57,3 et FP $UVA_{PPD}$ vivo = 20,9 (5 sujets)

| Ingrédients | Quantités % en poids |
|---|---|
| Phase A | |
| ETHYLENEDIAMINE/STEARYL DIMER DILINOLEATE COPOLYMER (UNICLEAR 100 VG - ARIZONA CHEMICAL ) | 6,0 |
| CAPRYLIC/CAPRIC TRIGLYCERIDE (60/40) (MYRITOL 318- COGNIS) | 33,0 |
| BUTYL METHOXYDIBENZOYLMETHANE | 5,0 |
| ETHYLHEXYL TRIAZONE | 5,0 |
| OCTOCRYLENE | 5,0 |

(suite)

| Ingrédients | Quantités % en poids |
|---|---|
| Phase A | |
| ETHYLHEXYL SALICYLATE | 5,0 |
| DROMETRIZOLE TRISILOXANE | 1,0 |
| DIETHYLHEXYL BUTAMIDO TRIAZONE (UVASORB HEB - SIGMA 3V) | 3,0 |
| C12-15 ALKYL BENZOATE (TEGOSOFT TN de EVONIK GOLDSCHMIDT) | 13,0 |
| OCTYL-2-DODECANOL | qsp 100 |
| Phase B | |
| ETHANOL | 8,0 |
| PARFUM | 0,3 |

Le mode opératoire est le suivant :

On prépare la phase A en mélangeant les matières premières et on chauffe à 90-95°C sous agitation jusqu'à solubilisation totale des matières premières. On refroidit à 25°C sous agitation. On incorpore ensuite la phase B à 25°C sous agitation jusqu'à parfaite homogénéisation. On évalue pour chacune des compositions

(1) le FPS in vivo sur 5 sujets selon la méthode Internationale publiée par Colipa / CTFA SA / JCIA (Mai 2006) ;
(2) le FP $UVA_{PPD}$ sur 5 sujets selon les recommandations du JCIA (version du 15/11/1995).

**Exemples 2 et 3**

On réalise les formulations 2 et 3 selon le même mode opératoire et on évalue pour chacune des compositions le FPS in vitro. On mesure le FPS moyen de chaque formulation selon la méthode d'évaluation du facteur de protection utilisée est la méthode in vitro décrite par B. L. DIFFEY et al. dans J. Soc. Cosmet Chem. 40-127-133 (1989), qui consiste à déterminer les facteurs de protection monochromatiques tous les 5 nm dans une gamme de longueurs d'onde de 290 à 400nm, et à calculer, à partir de ceux-ci, le facteur de protection solaire selon une équation mathématique donnée. Chaque composition testée est appliquée sur des plaques de quartz + Transpore® avec 5 plaques par essai et 4 mesures par plaque. L'appareil spectrophotomètre utilisé est Labsphere UV 1000S .

| Ingrédients | Ex2 % en poids | Ex3 (hors invention) % en poids |
|---|---|---|
| ETHYLENEDIAMINE/STEARYL DIMER DILINOLEATE COPOLYMER (UNICLEAR 100 VG - ARIZONA CHEMICAL ) | 6,0 | - |
| CAPRYLIC/CAPRIC TRIGLYCERIDE (60/40) (MYRITOL 318- COGNIS) | 33,0 | 33,0 |
| BUTYL METHOXYDIBENZOYLMETHANE | 3,5 | 3,5 |
| OCTOCRYLENE | 5,0 | 5,0 |
| ETHYLHEXYL SALICYLATE | 5,0 | 5,0 |
| DROMETRIZOLE TRISILOXANE | 1,0 | 1,0 |
| DIETHYLHEXYL BUTAMIDO TRIAZONE | 3,0 | 3,0 |
| C12-15 ALKYL BENZOATE (TEGOSOFT TN de EVONIK GOLDSCHMIDT) | 13,0 | 13,0 |
| ETHANOL | 3,0 | 3,0 |
| OCTYL-2-DODECANOL | qsp 100 | qsp 100 |
| **FPS IN VITRO** | **100,6** | **36,2** |

Ces résultats montrent clairement que l'ajout d'un agent rhéologique épaississant de phase grasse conduit à une forte augmentation du FPS.

**Exemples 4 à 6**

On réalise les formulations 4 à 6 selon le même mode opératoire et on évalue pour chacune des compositions l'écoulement selon le protocole suivant :

Des mesures sont réalisées à 25°C sur un appareil Haake RS600 muni d'une géométrie cône plan en titane sablé de 600mm 2 degré. Les mesures sont faites en écoulement à contrainte imposée, de 0.1 Pa à 100 Pa. L'évaluation de l'écoulement est basée sur les contraintes seuil qui correspondent à la rupture de pente sur les courbes de déformation en fonction de la contrainte.

On a construit ces compositions à partir de l'exemple 6 en substituant tout ou partie du filtre Ethylhexyl Triazone par le diethylhexyl butamido triazone, ces deux filtres ayant des propriétés filtrantes équivalentes.

| **Ingrédients** | **Ex4** | **Ex5** | **Ex6 (hors invention)** |
|---|---|---|---|
| ETHYLENEDIAMINE/STEARYL DIMER DILINOLEATE COPOLYMER (UNICLEAR 100 VG - ARIZONA CHEMICAL ) | 6,0 | 6,0 | 6,0 |
| CAPRYLIC/CAPRIC TRIGLYCERIDE (60/40) (MYRITOL 318-COGNIS) | 33,0 | 33,0 | 33,0 |
| BUTYL METHOXYDIBENZOYLMETHA NE | 3,5 | 3,5 | 3,5 |
| OCTOCRYLENE | 5,0 | 5,0 | 5,0 |
| ETHYLHEXYL SALICYLATE | 5,0 | 5,0 | 5,0 |
| DROMETRIZOLE TRISILOXANE | 1,0 | 1,0 | 1,0 |
| ETHYLHEXYL TRIAZONE | - | 3,0 | 6,0 |
| DIETHYLHEXYL BUTAMIDO TRIAZONE | 6,0 | 3,0 | - |
| C12-15 ALKYL BENZOATE (TEGOSOFT TN de EVONIK GOLDSCHMIDT) | 13,0 | 13,0 | 13,0 |
| ETHANOL | 5,0 | 5,0 | 5,0 |
| OCTYL-2-DODECANOL | qsp 100 | qsp 100 | qsp 100 |
| **Observation de l'écoulement** | **Bon écoulement** | **Ecoulement moyen** | **Ecoulement faible** |

Ces résultats montrent clairement qu'en conservant le pouvoir filtrant de la composition (substitution de l'Ethylhexyl Triazone par le Diethylhexyl Butamido Triazone), l'emploi du Diethylhexyl Butamido Triazone à la place d'un autre filtre triazine a amélioré les propriétés d'usage de la composition, notamment sa capacité à l'écouler.

D'autres exemples selon l'invention réalisés avec le mode opératoire ci-dessous mettant en oeuvre différents agents rhéologiques gélifiants d'huiles sont donnés ci-dessous à titre illustratif de l'invention :

| **Ingrédients** | **Ex7** | | **Ex8** |
|---|---|---|---|
| ETHYLENEDIAMINE/HYDRO GENATED DIMER DILINOLEATE COPOLYMER BIS-DI-C14-18 ALKYL AMIDE (UNICLEAR A200V - ARIZONA CHEMICAL ) | 6,0 | - | - |
| SYNTHETIC WAX (CIREBELLE 303 - SASOL) | - | | - |
| POLY C10-30 ALKYL ACRYLATE (INTELIMER IPA 13-6 -AIR PRODUCTS) | - | - | 5,0 |
| CAPRYLIC/CAPRIC TRIGLYCERIDE (60/40) (MYRITOL 318- COGNIS) | 33,0 | | 33,0 |
| BUTYL METHOXYDIBENZOYLMETH ANE | 3,5 | | 3,5 |

(suite)

| Ingrédients | Ex7 | | Ex8 |
|---|---|---|---|
| OCTOCRYLENE | 5,0 | | 5,0 |
| ETHYLHEXYL SALICYLATE | 5,0 | | 5,0 |
| DROMETRIZOLE TRISILOXANE | 1,0 | | 1,0 |
| DIETHYLHEXYL BUTAMIDO TRIAZONE | 5,0 | | 5,0 |
| C12-15 ALKYL BENZOATE (TEGOSOFT TN de EVONIK GOLDSCHMIDT) | 13,0 | | 13,0 |
| ETHANOL | 5,0 | | 5,0 |
| OCTYL-2-DODECANOL | qsp 100 | | qsp 100 |

## Revendications

**1.** Composition anhydre fluide comprenant dans un milieu cosmétiquement acceptable :

a) au moins une phase huileuse et
b) au moins un filtre UV triazine de formule (I) suivante :

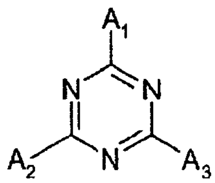

(I)

dans laquelle les radicaux $A_1$, $A_2$ et $A_3$, identiques ou différents désigne un groupe de formule (II) suivante :

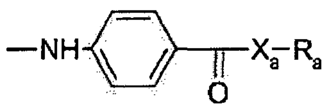

(II)

- un ou deux des groupements $X_a$-$R_a$ représentent le radical -NH-$R_a$ avec $R_a$ choisi parmi : un radical alkyle linéaire ou ramifié en $C_1$-$C_{18}$, un radical cycloalkyle en $C_5$-$C_{12}$ éventuellement substitué avec un ou plusieurs radicaux alkyles en $C_1$-$C_4$; un radical de formule ($\alpha$), ($\beta$) ou ($\varepsilon$) suivantes :

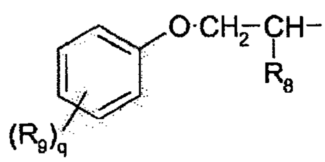

($\alpha$)

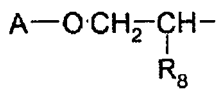

($\beta$)

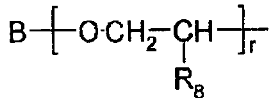

(ε)

dans lesquelles :

- $R_8$ est l'hydrogène ou un radical alkyle en $C_1$-$C_4$ de préférence méthyle ;
- $R_9$ est alkyle en $C_1$-$C_4$ et de préférence méthyle ;
- q est un nombre entier de 0 à 3;
- r est un nombre entier de 1 à 10;
- A est un radical alkyle en $C_4$-$C_8$ ou un radical cycloalkyle en $C_5$-$C_8$ ;
- B est un radical alkyle en $C_1$-$C_4$ ;

- le ou les deux autres $X_a$-$R_a$ étant le radical -O-$R_a$ avec $R_a$, identiques ou différents choisis parmi: l'hydrogène; un métal alcalin; un radical ammonium éventuellement substitué par un ou plusieurs radicaux alkyles ou hydroxyalkyles ; un radical alkyle linéaire ou ramifié en $C_1$-$C_{18}$, un radical cycloalkyle en $C_5$-$C_{12}$ éventuellement substitué par un ou plusieurs radicaux alkyles en $C_1$-$C_4$ ; un radical de formule ($\alpha$), ($\beta$) ou ($\varepsilon$) telles que définies ci-dessus ;

c) au moins un agent rhéologique épaississant ou gélifiant de phase huileuse choisi parmi :

(i) les polymères semi-cristallins issus d'un monomère à chaîne cristallisable choisi parmi les (méth)acrylates d'alkyle saturés en $C_{14}$ à $C_{22}$, et
(ii) les polycondensats polyamides lipophiles.

**2.** Composition selon la revendication 1, où le composé de formule (I) est la 2-[(p-(tertiobutylamido)anilino]-4,6-bis-[(p-(2'-éthylhexyl-1'-oxycarbonyl)anilino]-1,3,5-triazine ou «Diethylhexyl Butamido Triazone» répondant à la formule suivante :

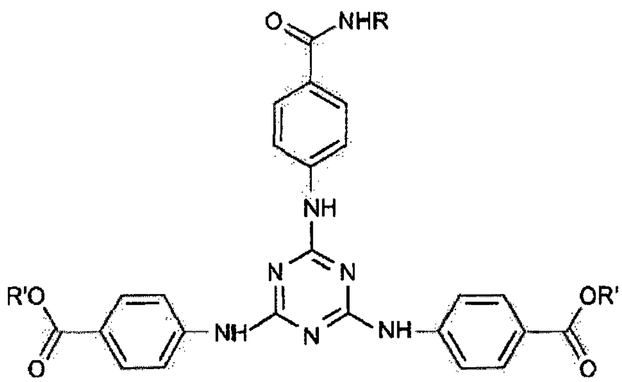

dans laquelle R' désigne un radical éthyl-2 hexyle et R désigne un radical tert-butyle.

**3.** Composition selon la revendication 1 ou 2, où le composé triazine de formule (I) est présent dans des proportions allant de 0,1 à 10% en poids par rapport au poids total de la composition, et de préférence allant de 1 à 6% en poids par rapport au poids total de la composition.

**4.** Composition selon l'une quelconque des revendications 1 à 3, où l'agent rhéologique est présent en des quantités allant de 0.1% à 10%, plus préférentiellement de 1% à 7%, et encore plus préférentiellement de 4% à 7% par rapport au poids total de la composition.

**5.** Composition selon l'une des revendications 1 à 4, où l'agent rhéologique épaississant ou gélifiant de phase huileuse (i) est choisi parmi les poly(acrylate de stéaryle) ou les poly(acrylate de béhényle).

**6.** Composition selonl'une des revendications 1 à 4, où l'agent rhéologique épaississant ou gélifiant de phase huileuse est un polycondensat polyamide lipophile.

**7.** Composition selon l'une quelconque des revendications 1 à 6, où la phase huileuse représente une quantité de 30 à 99,8% en poids et de préférence de 40 à 90% en poids par rapport au poids total de la composition.

**8.** Composition selon l'une quelconque des revendications 1 à 7, comprenant en outre au moins un mono-alcool en $C_1$-$C_3$ en particulier l'éthanol.

**9.** Composition selon l'une quelconque des revendications 1 à 8, comprenant en outre au moins un filtre organique lipophile additionnel.

**10.** Composition selon l'une quelconque des revendications 1 à 9, où le ou les filtres organiques lipophiles sont choisis parmi les dérivés de l'acide para-aminobenzoique, les dérivés salicyliques, les dérivés cinnamiques, les benzophénones et aminobenzophénones, les dérivés anthraniliques, les dérivés du dibenzoylméthane, les dérivés de β,β-diphénylacrylate, les dérivés du benzylidène camphre, les dérivés du phényl benzimidazole, les dérivés de benzotriazole, les dérivés triazine autres que ceux de formule (I), les bis-résorcinyl triazines, les dérivés d'imidazolines, les dérivés du benzalmalonate, les dérivés de 4,4-diarylbutadiène, les dérivés de benzoxazole, les mérocyanines et leurs mélanges.

**11.** Composition selon l'une quelconque des revendications 1 à 10, **caractérisée par le fait qu'**elle est transparente et présente une turbidité inférieure à 1000 NTU à 25°C, de préférence inférieure à 50 NTU à 25°C et encore plus préférentiellement inférieure à 15 NTU.

**12.** Composition selon l'une quelconque des revendications 1 à 11, **caractérisée par le fait qu'**elle présente un FPS supérieur à 10, voire supérieur à 20 et même supérieur à 30.

**13.** Composition selon l'une quelconque des revendications 1 à 12, **caractérisée par le fait qu'**elle présente un FP $UVA_{PPD}$ supérieur à 5, voire supérieur à 7 et même supérieur à 10 et/ou un ratio SPF /PPD inférieur à 3.

**14.** Composition telle que définie dans l'une quelconque des revendications 1 à 13 comme produit pour le soin cosmétique et/ou le maquillage et/ou de la peau, des ongles, des cheveux, des cils, sourcils et/ou du cuir chevelu.

## Patentansprüche

**1.** Wasserfreie fließfähige Zusammensetzung, umfassend in einem kosmetisch unbedenklichen Medium:

a) mindestens eine Ölphase und
b) mindestens ein Triazin-UV-Filter der folgenden Formel (I):

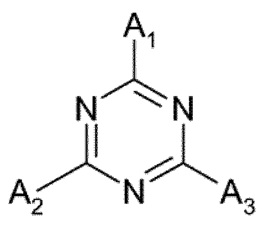

(I)

in der die Reste $A_1$, $A_2$ und $A_3$ gleich oder verschieden sind und für eine Gruppe der folgenden Formel (II) stehen:

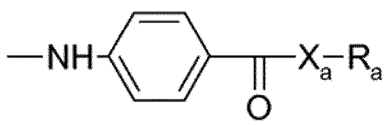

(II)

- eine oder zwei der Gruppen $X_a$-$R_a$ für den Rest -NH-$R_a$ stehen, wobei $R_a$ aus einem linearen oder verzweigten $C_1$-$C_{18}$-Alkylrest; einem $C_5$-$C_{12}$-Cycloalkylrest, der gegebenenfalls durch einen oder mehrere

$C_1$-$C_4$-Alkylreste substituiert ist; und einem Rest der folgenden Formel (α), (β) oder (ε):

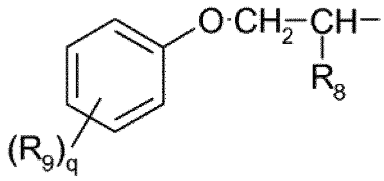

(α)

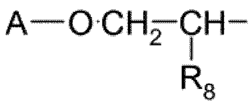

(β)

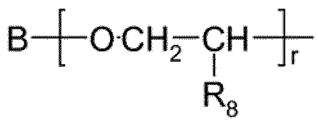

(ε)

in denen:

- $R_8$ für Wasserstoff oder einen $C_1$-$C_4$-Alkylrest, vorzugsweise Methyl, steht;
- $R_9$ für $C_1$-$C_4$-Alkyl und vorzugsweise Methyl steht;
- q für eine ganze Zahl von 0 bis 3 steht;
- r für eine ganze Zahl von 1 bis 10 steht;
- A für einen $C_4$-$C_8$-Alkylrest oder einen $C_5$-$C_8$-Cycloalkylrest steht;
- B für einen $C_1$-$C_4$-Alkylrest steht;

ausgewählt ist;
- wobei das $X_a$-$R_a$ bzw. die beiden anderen $X_a$-$R_a$ für den Rest -O-$R_a$ stehen, wobei die Reste $R_a$ gleich oder verschieden sind und aus Wasserstoff; einem Alkalimetall; einem Ammoniumrest, der gegebenenfalls durch einen oder mehrere Alkyl- oder Hydroxyalkylreste substituiert ist; einem linearen oder verzweigten $C_1$-$C_{18}$-Alkylrest; einem $C_5$-$C_{12}$-Cycloalkylrest, der gegebenenfalls durch einen oder mehrere $C_1$-$C_4$-Alkylreste substituiert ist; und einem Rest der Formel (α), (β) oder (ε) gemäß obiger Definition ausgewählt sind;

c) mindestens ein die Ölphase verdickendes oder gelierendes Rheologiemittel ausgewählt aus:

(i) teilkristallinen Polymeren aus einem Monomer mit kristallisierbarer Kette, das aus gesättigten $C_{14}$- bis $C_{22}$-Alkyl (meth) - acrylaten ausgewählt ist, und
(ii) lipophilen Polyamid-Polykondensaten.

**2.** Zusammensetzung nach Anspruch 1, wobei es sich bei der Verbindung der Formel (I) um 2-[(p-(tert.-Butylamido)anilino]-4,6-bis[(p-(2'-ethylhexyl-1'-oxycarbonyl)anilino]-1,3,5-triazin oder "Diethylhexyl Butamido Triazone" der folgenden Formel:

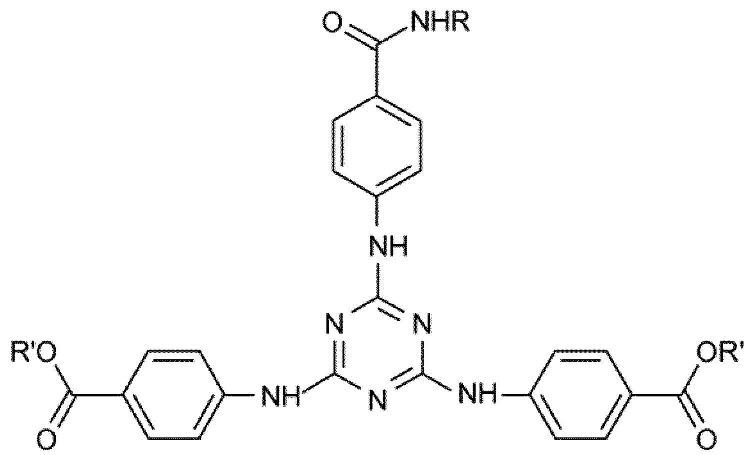

in der R' für einen 2-Ethylhexylrest steht und R für einen tert.-Butylrest steht, handelt.

**3.** Zusammensetzung nach Anspruch 1 oder 2, wobei die Triazinverbindung der Formel (I) in Anteilen im Bereich von 0,1 bis 10 Gew.-%, bezogen auf das Gesamtgewicht der Zusammensetzung, und vorzugsweise im Bereich von 1 bis 6 Gew.-%, bezogen auf das Gesamtgewicht der Zusammensetzung, vorliegt.

**4.** Zusammensetzung nach einem der Ansprüche 1 bis 3, wobei das Rheologiemittel in Mengen im Bereich von 0,1% bis 10%, weiter bevorzugt 1% bis 7% und noch weiter bevorzugt 4% bis 7%, bezogen auf das Gesamtgewicht der Zusammensetzung, vorliegt.

**5.** Zusammensetzung nach einem der Ansprüche 1 bis 4, wobei das die Ölphase verdickende oder gelierende Rheologiemittel (i) aus Poly(stearylacrylat) oder Poly(behenylacrylat) ausgewählt ist.

**6.** Zusammensetzung nach einem der Ansprüche 1 bis 4, wobei es sich bei dem die Ölphase verdickenden oder gelierenden Rheologiemittel um ein lipophiles Polyamid-Polykondensat handelt.

**7.** Zusammensetzung nach einem der Ansprüche 1 bis 6, wobei die Ölphase eine Menge von 30 bis 99,8 Gew.-% und vorzugsweise von 40 bis 90 Gew.-%, bezogen auf das Gesamtgewicht der Zusammensetzung, ausmacht.

**8.** Zusammensetzung nach einem der Ansprüche 1 bis 7, die außerdem mindestens einen $C_1$-$C_3$-Monoalkohol, insbesondere Ethanol, umfasst.

**9.** Zusammensetzung nach einem der Ansprüche 1 bis 8, die außerdem mindestens ein zusätzliches lipophiles organisches Filter umfasst.

**10.** Zusammensetzung nach einem der Ansprüche 1 bis 9, wobei das oder die lipophilen organischen Filter aus para-Aminobenzoesäurederivaten, Salicylsäurederivaten, Zimtsäurederivaten, Benzophenonen und Aminobenzophenonen, Anthranilsäurederivaten, Dibenzoylmethanderivaten, β,β-Diphenylacrylatderivaten, Benzylidencampherderivaten, Phenylbenzimidazolderivaten, Benzotriazolderivaten, Triazinderivaten, die von denjenigen der Formel (I) verschieden sind, Bisresorcinyltriazinen, Imidazolinderivaten, Benzalmalonatderivaten, 4,4-Diarylbutadienderivaten, Benzoxazolderivaten, Merocyaninderivaten und Mischungen davon ausgewählt sind.

**11.** Zusammensetzung nach einem der Ansprüche 1 bis 10, **dadurch gekennzeichnet, dass** sie transparent ist und eine Trübung von weniger als 1000 NTU bei 25°C, vorzugsweise weniger als 50 NTU bei 25°C und noch weiter bevorzugt weniger als 15 NTU aufweist.

**12.** Zusammensetzung nach einem der Ansprüche 1 bis 11, **dadurch gekennzeichnet, dass** sie einen LSF von mehr als 10, sogar mehr als 20 und sogar mehr als 30 aufweist.

**13.** Zusammensetzung nach einem der Ansprüche 1 bis 12, **dadurch gekennzeichnet, dass** sie einen $UVA_{PPD}$-SF von mehr als 5, sogar mehr als 7 und sogar mehr als 10 und/oder ein LSF/PPD-Verhältnis von weniger als 3 aufweist.

**14.** Zusammensetzung gemäß einem der Ansprüche 1 bis 13 als Produkt für die kosmetische Pflege und/oder das Schminken und/oder für die Haut, die Nägel, die Haare, die Wimpern, die Augenbrauen und/oder die Kopfhaut.

## Claims

**1.** Anhydrous fluid composition comprising, in a cosmetically acceptable medium:

a) at least one oily phase and
b) at least one triazine UV filter of the following formula (I):

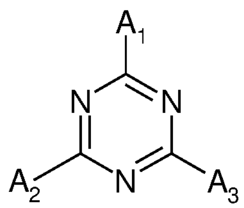

(I)

in which the radicals $A_1$, $A_2$ and $A_3$, which may be identical or different, denote a group of the following formula (II):

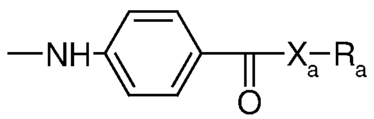

(II)

- one or two of the groups $X_a$-$R_a$ represent the radical -NH-$R_a$ with $R_a$ selected from: a linear or branched $C_1$-$C_{18}$ alkyl radical; a $C_5$-$C_{12}$ cycloalkyl radical optionally substituted with one or more $C_1$-$C_4$ alkyl radicals; a radical of the following formula (α), (β) or (ε) :

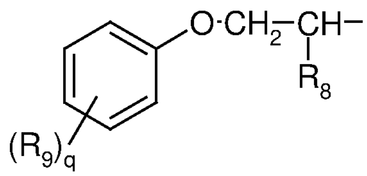

(α)

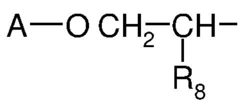

(β)

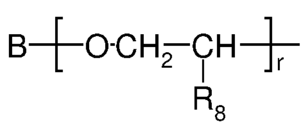

(ε)

in which:

- $R_8$ is hydrogen or a $C_1$-$C_4$ alkyl radical, preferably methyl;

- $R_9$ is $C_1$-$C_4$ alkyl and preferably methyl;
- q is an integer from 0 to 3;
- r is an integer from 1 to 10;
- A is a $C_4$-$C_8$ alkyl radical or a $C_5$-$C_8$ cycloalkyl radical;
- B is a $C_1$-$C_4$ alkyl radical;
- the other two $X_a$-$R_a$ being the radical -O-$R_a$ with $R_a$, which may be identical or different, selected from: hydrogen; an alkali metal; an ammonium radical optionally substituted with one or more alkyl or hydroxyalkyl radicals; a linear or branched $C_1$-$C_{18}$ alkyl radical; a $C_5$-$C_{12}$ cycloalkyl radical optionally substituted with one or more $C_1$-$C_4$ alkyl radicals; a radical of formula (α), (β) or (ε) as defined above;

c) at least one rheological agent for thickening or gelling the oily phase, selected from:

(i) semicrystalline polymers derived from a monomer with a crystallizable chain selected from saturated $C_{14}$ to $C_{22}$ alkyl (meth) acrylates, and
(ii) lipophilic polyamide polycondensates.

**2.** Composition according to Claim 1, where the compound of formula (I) is 2-[(p-(tert-butylamido)anilino]-4,6-bis-[(p-(2'-ethylhexyl-1'-oxycarbonyl)anilino]-1,3,5-triazine or "Diethylhexyl Butamido Triazone" corresponding to the following formula:

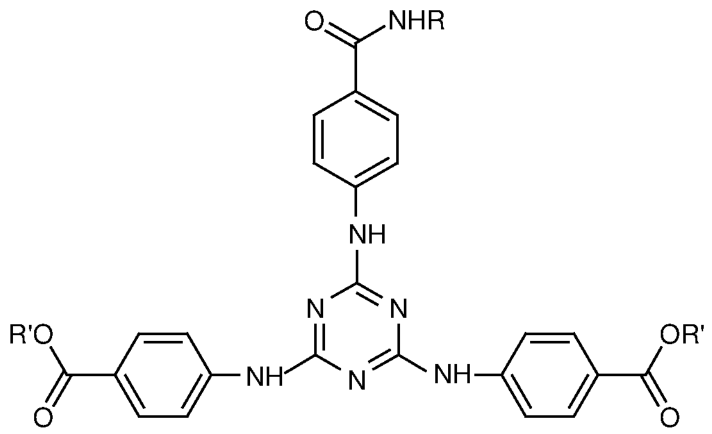

in which R' denotes an ethyl-2-hexyl radical and R denotes a tert-butyl radical.

**3.** Composition according to Claim 1 or 2, where the triazine compound of formula (I) is present in proportions in the range from 0.1 to 10 wt.% relative to the total weight of the composition, and preferably in the range from 1 to 6 wt.% relative to the total weight of the composition.

**4.** Composition according to any one of Claims 1 to 3, where the rheological agent is present in amounts in the range from 0.1% to 10%, more preferably from 1% to 7%, and even more preferably from 4% to 7% relative to the total weight of the composition.

**5.** Composition according to one of Claims 1 to 4, where the rheological agent (i) for thickening or gelling the oily phase is selected from poly(stearyl acrylate) or poly(behenyl acrylate).

**6.** Composition according to one of Claims 1 to 4, where the rheological agent for thickening or gelling the oily phase is a lipophilic polyamide polycondensate.

**7.** Composition according to any one of Claims 1 to 6, where the oily phase represents an amount from 30 to 99.8 wt.% and preferably from 40 to 90 wt.% relative to the total weight of the composition.

**8.** Composition according to any one of Claims 1 to 7, further comprising at least one $C_1$-$C_3$ monohydric alcohol, in particular ethanol.

**9.** Composition according to any one of Claims 1 to 8, further comprising at least one additional lipophilic organic filter.

**10.** Composition according to any one of Claims 1 to 9, where the lipophilic organic filter(s) are selected from derivatives of para-aminobenzoic acid, salicylic derivatives, cinnamic derivatives, benzophenones and aminobenzophenones, anthranilic derivatives, derivatives of dibenzoylmethane, derivatives of β,β-diphenylacrylate, derivatives of benzylidene camphor, derivatives of phenyl benzimidazole, derivatives of benzotriazole, triazine derivatives other than those of formula (I), bis-resorcinyl triazines, derivatives of imidazolines, derivatives of benzalmalonate, derivatives of 4,4-diarylbutadiene, derivatives of benzoxazole, merocyanines and mixtures thereof.

**11.** Composition according to any one of Claims 1 to 10, **characterized in that** it is transparent and has a turbidity less than 1000 NTU at 25°C, preferably less than 50 NTU at 25°C and even more preferably less than 15 NTU.

**12.** Composition according to any one of Claims 1 to 11, **characterized in that** it has an SPF greater than 10, or greater than 20 and even greater than 30.

**13.** Composition according to any one of Claims 1 to 12, **characterized in that** it has a $UVA_{PPD}$ PF greater than 5, or greater than 7 and even greater than 10 and/or an SPF/PPD ratio of less than 3.

**14.** Composition as defined in any one of Claims 1 to 13 as product for cosmetic care and/or make-up and/or for the skin, nails, hair, eyelashes, eyebrows and/or scalp.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9906014 A
- EP 845261 A
- EP 845260 A
- EP 821938 A
- EP 821940 A
- EP 800813 A
- EP 1300137 A
- EP 1027883 A
- EP 1813266 A
- EP 2014277 A
- US 5783657 A
- US 6503522 B
- US 6552160 B
- US 6399713 B
- US 3645705 A
- US 3148125 A
- US 5500209 A
- US 5998570 A
- WO 2007068371 A
- WO 20081155059 A
- US 4077441 A
- US 4850517 A


### Littérature non-brevet citée dans la description


- méthode Internationale. Colipa / CTFA SA / JCIA, Mai 2006
- **B. L. DIFFEY et al.** *J. Soc. Cosmet Chem.,* 1989, vol. 40, 127-133